# FASCICULE DE BREVET EUROPEEN

(11) **EP 2 459 099 B1**
(45) Date de publication et mention de la délivrance du brevet: **06.06.2018**
(21) Numéro de dépôt: 10745426.6
(22) Date de dépôt: 30.07.2010
(51) Int. Cl.: A61B 17/29, A61B 34/00, A61B 17/04

(54) **MANIPULATEUR A TENUE MANUELLE ET ARTICULATION DE CONFORT**
MANIPULATOR MIT MANUELLER HALTERUNG UND KOMFORTABLER GELENKIGKEIT
MANIPULATOR WITH MANUAL HOLD AND COMFORTABLE ARTICULATION

(30) Priorité: 31.07.2009 FR 0955383
(43) Date de publication de la demande: 06.06.2012
(73) Titulaire: Dex Surgical, 91370 Verrières-le-Buisson (FR)
(72) Inventeur: BARRIER, Pascal, 74000 Annecy (FR); OLLAGNIER, Jérémy, 74960 Meythet (FR); ROSSET-LANCHET, Rémi, 74000 Annecy (FR)
(74) Mandataire: Cabinet Poncet
(86) Numéro de dépôt international: PCT/IB2010/053475
(87) Numéro de publication internationale: WO 2011/013102

(56) Documents cités:
- DE-U1- 9 203 041
- DE-U1- 29 623 921
- NL-C2- 1 004 375
- US-A1- 2005 222 587
- US-A1- 2006 020 287
- US-A1- 2008 287 862

## Description

### DOMAINE TECHNIQUE DE L'INVENTION

La présente invention concerne les dispositifs de manipulation permettant de commander, depuis l'extérieur d'une zone d'intervention, les mouvements d'un instrument de manipulation situé à l'intérieur de la zone d'intervention.

En particulier, ces dispositifs de manipulation permettent de placer et commander un outil chirurgical dans les applications de chirurgie mini-invasive pratiquées sous endoscopie.

Dans ces applications, il est nécessaire de pouvoir déplacer et commander un outil chirurgical pour effectuer diverses opérations telles qu'une suture, le serrage d'un noeud, une fine dissection de tissus. Il s'agit alors d'opérations délicates, précises, dans lesquelles les mouvements à réaliser sont complexes.

L'invention concerne plus spécialement les dispositifs portables de manipulation, dans lesquels un outil chirurgical est placé en bout d'un manipulateur que le chirurgien porte par une seule main et ayant essentiellement un bras de liaison à extrémité proximale et extrémité distale. L'extrémité proximale du bras de liaison comporte une unité de commande ayant une poignée apte à être tenue par ladite main et comportant au moins un organe de commande apte à être sollicité par au moins un doigt de ladite main tenant la poignée pour actionner l'instrument chirurgical.

On connaît par exemple du document US 6,077,286, un tel dispositif portable de manipulation, dans lequel la poignée est une tige munie d'un levier se développant tout le long de la tige et articulé à son extrémité distale, de façon que la main de l'utilisateur tenant la poignée puisse faire pivoter le levier vers l'ouverture et la fermeture tout en portant le manipulateur.

Le levier constitue ainsi un organe de commande monté sur la poignée et apte à être sollicité par au moins un doigt de la main tenant la poignée.

La poignée est montée à l'extrémité proximale d'un bras de liaison qui se développe le long d'un axe longitudinal de bras et qui porte, à son extrémité distale, une unité de travail portant un outil. L'outil peut avoir des mâchoires, dont l'ouverture et la fermeture sont commandées par le pivotement du levier.

Ce document propose d'améliorer l'ergonomie de tenue et manipulation de la poignée en prévoyant une liaison articulée selon un axe transversal entre l'extrémité proximale du bras de liaison et l'extrémité distale de la poignée, avec des moyens de blocage en position angulaire de la poignée. Ainsi, dans ce document, l'articulation de la poignée est à l'extrémité distale de la poignée, et le tronçon de préhension selon lequel l'utilisateur tient la poignée est entièrement en zone proximale par rapport à l'articulation.

Dans le dispositif de ce document antérieur, la sollicitation du levier articulé produit nécessairement une sollicitation simultanée sur la poignée, de sorte qu'il n'y a pas un découplage parfait entre le mouvement de fermeture ou d'ouverture de l'outil par la rotation du levier et le positionnement de l'outil en bout du bras de liaison.

En outre, lorsqu'on a pivoté la poignée, pour améliorer le confort de l'opérateur, une traction ou une poussée sur la poignée pour réaliser un mouvement axial du manipulateur est excentrée par rapport à l'axe du bras de liaison, ce qui produit nécessairement un couple d'inclinaison du manipulateur, et un déplacement intempestif de l'outil, que l'opérateur doit tenter de corriger en appliquant un couple sur la poignée.

En outre, dans la position pivotée de la poignée, une sollicitation en traction sur la poignée pour réaliser un mouvement axial du manipulateur produit nécessairement une sollicitation parasite sur le levier et donc un actionnement intempestif de l'outil.

Simultanément, on cherche à réaliser une meilleure ergonomie du manipulateur portable, permettant de tenir le manipulateur et de solliciter l'outil chirurgical dans tous les mouvements désirés sans que l'opérateur n'ait à placer sa main dans des positions limites ou inconfortables de l'articulation de son poignet, de son coude et de son épaule.

Le document US 2008/0287862 A1 décrit un manipulateur comprenant :
- une unité de commande, ayant une poignée apte à être tenue par une main, et ayant des moyens de fixation et de guidage pour sa fixation à coulissement sur un support fixe,
- au moins un organe de commande, monté sur la poignée, et apte à être sollicité par au moins un doigt de la main tenant la poignée,
- un bras de liaison, se développant le long d'un axe longitudinal de bras, ayant une extrémité proximale dans laquelle est montée l'unité de commande, et ayant une extrémité distale,
- une unité de travail, montée sur l'extrémité distale du bras de liaison, comportant un support d'outils apte à supporter un outil, et actionnée par ledit au moins un organe de commande,
- la poignée étant allongée le long d'un axe longitudinal de poignée et étant conformée pour être tenue dans la paume de la main avec le pouce en opposition des autres doigts pour entourer sa surface latérale selon un tronçon de préhension,
- la poignée étant articulée, dans l'unité de commande, selon au moins un degré de liberté de rotation autour d'un premier axe d'articulation transversal par rapport à son axe longitudinal de poignée,
- le premier axe d'articulation de la poignée étant en position intermédiaire le long de l'axe longitudinal de poignée, de sorte que le tronçon de préhension de la poignée s'étend de part et d'autre du premier axe d'articulation de la poignée,
- le premier axe d'articulation étant sécant avec l'axe longitudinal du bras de liaison.

Grâce à la position intermédiaire du premier axe d'articulation de la poignée, et grâce au fait que l'axe d'articulation est sécant avec l'axe longitudinal du bras de liaison, l'opérateur tient la poignée de part et d'autre du premier axe d'articulation de la poignée, et les mouvements de traction ou de poussée axiale sur le manipulateur produisent des couples sensiblement opposés de rotation de la poignée, réduisant les éventuels efforts parasites appliqués latéralement sur le manipulateur dans un plan perpendiculaire au premier axe d'articulation. On constate toutefois qu'il reste encore des efforts parasites dans les plans non perpendiculaires au premier axe d'articulation, de sorte que la précision du positionnement et de l'entraînement de l'outil n'est pas encore suffisante.

En outre, dans ce document, les mouvements relatifs de la poignée par rapport à l'unité de commande provoquent la sollicitation d'actionneurs qui déplacent l'outil distal par rapport au bras de liaison. Cela induit une non dissociation entre la mise en position de l'outil et la commande des mouvements de l'outil lors d'une intervention chirurgicale. Pour cela, le document prévoit la fixation de l'unité de commande sur un support fixe. Le manipulateur n'est pas portable.

NL1004375C2 divulgue un manipulateur avec une poignée articulée autour d'une axe d'articulation transversal.

### EXPOSE DE L'INVENTION

Le problème proposé par la présente invention est d'améliorer encore la précision du positionnement et de l'entraînement en mouvement d'un outil par un manipulateur portable, en évitant tous les mouvements parasites et en assurant une bonne dissociation entre la mise en position de l'outil et la commande des mouvements de cet outil lors d'une intervention chirurgicale.

Simultanément, on cherche à permettre une rotation axiale aisée du manipulateur autour de l'axe longitudinal du bras de liaison par la seule manoeuvre de la poignée, en limitant l'amplitude des mouvements nécessaires du poignet, du coude et de l'épaule de l'opérateur.

Pour atteindre ces buts ainsi que d'autres, l'axe longitudinal de poignée est également sécant avec l'axe longitudinal du bras de liaison.

De la sorte, l'équilibrage des couples de rotation est assuré dans toutes les directions autour de l'axe longitudinal du bras de liaison.

En outre, la poignée est fixe en rotation axiale autour de son axe longitudinal par rapport à l'unité de commande, et le manipulateur est dépourvu de tout moyen de couplage direct ou indirect entre les mouvements relatifs de la poignée par rapport à l'unité de commande et les mouvements relatifs du support d'outils et de l'outil par rapport au bras de liaison.

L'absence de rotation relative de la poignée autour de son axe longitudinal permet d'éviter l'impossibilité ou la grande difficulté d'entraîner en rotation axiale le manipulateur lorsque la poignée se trouve sensiblement alignée avec le bras de liaison.

L'absence de tout moyen de couplage entre les mouvements relatifs de la poignée et les mouvements relatifs du support d'outils permet une bonne dissociation entre le maintien en position de l'outil et la commande des mouvements relatifs de l'outil, et permet que la main de l'opérateur maintienne aisément la poignée quelle que soit la position du manipulateur, et ce malgré les limitations physiologiques de la main humaine.

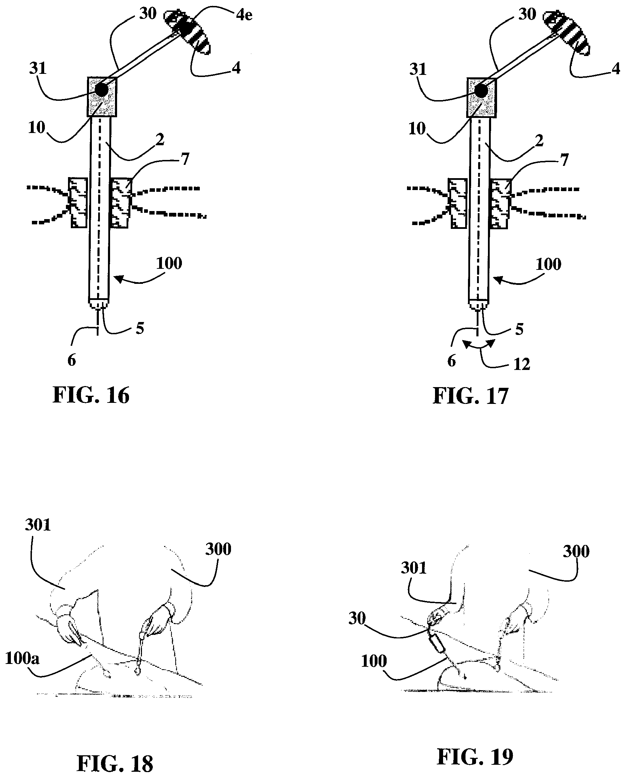

En outre, par la combinaison avec la position transversale du premier axe d'articulation par rapport à l'axe longitudinal de la poignée, l'amplitude des mouvements nécessaires du bras de l'opérateur pour faire pivoter le manipulateur autour de son axe longitudinal est fortement réduite, en particulier dans les positions déportées du manipulateur (figure 18).

Dans la description et les revendications, les expressions « sécant avec » doivent être considérées non pas dans un sens mathématique strict de droites sécantes qui se touchent en un point, mais plutôt dans un sens physique, plus approximatif procurant un effet technique suffisant de dissociation entre les différents mouvements de positionnement du manipulateur. Ainsi, on admettra que les trois axes considérés, à savoir le premier axe d'articulation, l'axe longitudinal de poignée et l'axe longitudinal du bras de liaison, sont « sécants » lorsqu'ils coupent tous les trois une sphère de 15 mm de diamètre. De la sorte, les bras de levier sont suffisamment faibles pour que les couples parasites induits par les différents mouvements soient négligeables. On admettra aussi que des axes sécants l'un avec l'autre peuvent être confondus (superposés) dans certaines positions.

Une amélioration est encore obtenue en prévoyant que le premier axe d'articulation est en position intermédiaire équilibrée le long du tronçon de préhension de la poignée. On s'assure de la sorte que les couples de rotation appliqués sur la poignée lors d'une traction ou d'une poussée axiale, ou lors d'une rotation de la poignée autour du premier axe, se compensent exactement, évitant toute sollicitation latérale sur le manipulateur, et évitant par conséquent tout déplacement intempestif de l'outil.

De préférence, le ou les organes de commande sont disposés sur la poignée à l'écart des zones d'appui du tronçon de préhension. Autrement dit, les organes de commande sont placés sur la poignée de telle sorte qu'ils soient sollicités par une partie de la main non utile à la préhension du manipulateur. On réduit ainsi encore les risques d'interférence entre les mouvements de positionnement de l'outil et les mouvements d'actionnement de l'outil.

Pour augmenter également les capacités de mouvements de la poignée, on peut prévoir que la poignée est en outre articulée dans l'unité de commande selon un second degré de liberté de rotation autour d'un second axe d'articulation.

Avantageusement, les axes d'articulation pourront être concourants avec l'axe longitudinal du bras.

Selon un premier mode de réalisation, la poignée est libre en pivotement dans l'unité de commande.

Selon un second mode de réalisation, on peut prévoir en outre des moyens de blocage de la poignée en rotation autour du ou des axes d'articulation, donnant ainsi à l'opérateur quelques possibilités supplémentaires de tenue du manipulateur si le bras de liaison est mal ou pas guidé dans un trocart.

On pourra toutefois préférer une structure plus simple dans laquelle la poignée est articulée selon un seul degré de liberté de rotation, autour du premier axe d'articulation. L'apprentissage de l'utilisation d'une telle structure est plus aisé, pour assurer un bon positionnement de l'outil dans la cavité opératoire et éviter les mouvements parasites.

Dans ce cas, il sera avantageux que le premier axe d'articulation soit oblique par rapport à l'axe longitudinal du bras de liaison : cette orientation oblique permet de mieux s'affranchir des limites physiologiques de la main humaine lors de la rotation du manipulateur autour de l'axe longitudinal du bras de liaison.

Dans une autre réalisation, on prévoit, entre l'articulation de la poignée et le corps de commande, un bras de positionnement, par lequel l'opérateur peut modifier et fixer la position angulaire et/ou spatiale relative du premier axe d'articulation de la poignée dans l'unité de commande.

Ce bras de positionnement peut être une tige semi-rigide de 5 à 30 cm de long, déformable sous des forces de flexion ou de torsion plus importantes que les seules forces produites par le poids du manipulateur et la résistance des tissus traités par le manipulateur. De la sorte, l'opérateur peut tenir le manipulateur sans que le bras déformable se déforme, et il peut à volonté déformer le bras par une sollicitation plus intense avec les deux mains. L'opérateur peut ainsi placer l'axe d'articulation de poignée dans tout l'espace, à son gré, pour une meilleure maniabilité et un confort amélioré du coude et/ou de l'épaule de l'opérateur.

En alternative, le bras de positionnement peut être une tige, portant à son extrémité proximale le premier axe d'articulation de la poignée, et articulée à son extrémité distale sur le bras de liaison ou un corps de commande par une articulation blocable.

La présence d'un tel bras de positionnement permet en réalité à l'opérateur de modifier et fixer, dans une large plage de possibilités, la position et l'orientation relative de la poignée par rapport au bras de liaison et au reste du manipulateur. L'opérateur peut ainsi conformer le manipulateur pour lui donner, avant d'effectuer un geste opératoire, une forme mieux adaptée au geste qu'il devra réaliser, de façon à réaliser le geste sans avoir à placer sa main dans des positions limites ou inconfortables de l'articulation de son poignet, de son coude ou de son épaule. Un tel résultat peut être obtenu indépendamment de la présence ou de l'absence d'une articulation de poignée.

La présence d'un tel bras de positionnement entre la poignée et le bras de liaison constitue une invention indépendante.

L'invention s'applique avantageusement au cas où l'actionnement d'un premier organe de commande commande un actionneur d'inclinaison logé dans un corps de commande, qui entraîne le support d'outils en un mouvement d'inclinaison.

### DESCRIPTION SOMMAIRE DES DESSINS

D'autres objets, caractéristiques et avantages de la présente invention ressortiront de la description suivante de modes de réalisation particuliers, faite en relation avec les figures jointes, parmi lesquelles :
- la figure 1 est une vue schématique générale illustrant un manipulateur selon un mode de réalisation de la présente invention, en position d'utilisation pour une opération chirurgicale ;
- les figures 2, 3 et 4 sont des vues en perspective illustrant une main tenant une poignée du manipulateur de la figure 1 ;
- les figures 5 et 6 sont des vues en perspective illustrant l'articulation d'une poignée de manipulateur selon un premier mode de réalisation de l'invention ;
- les figures 7 à 10 illustrent, en perspective, une articulation de poignée de manipulateur selon un second mode de réalisation de la présente invention, en quatre positions successives lors de la rotation axiale du manipulateur ;
- les figures 11, 12 et 13 illustrent une articulation de poignée de manipulateur selon un troisième mode de réalisation de la présente invention ;
- la figure 14 illustre en vue de côté un manipulateur ayant un bras de positionnement déformable et une poignée articulée ;
- la figure 15 illustre un manipulateur à bras de positionnement déformable et poignée fixe ;
- la figure 16 illustre une variante de manipulateur à bras de positionnement articulé et poignée articulée ;
- la figure 17 illustre un manipulateur à bras de positionnement articulé et poignée fixe ; et
- les figures 18 et 19 illustrent un effet ergonomique d'un manipulateur à bras de positionnement.

### DESCRIPTION DES MODES DE REALISATION PREFERES

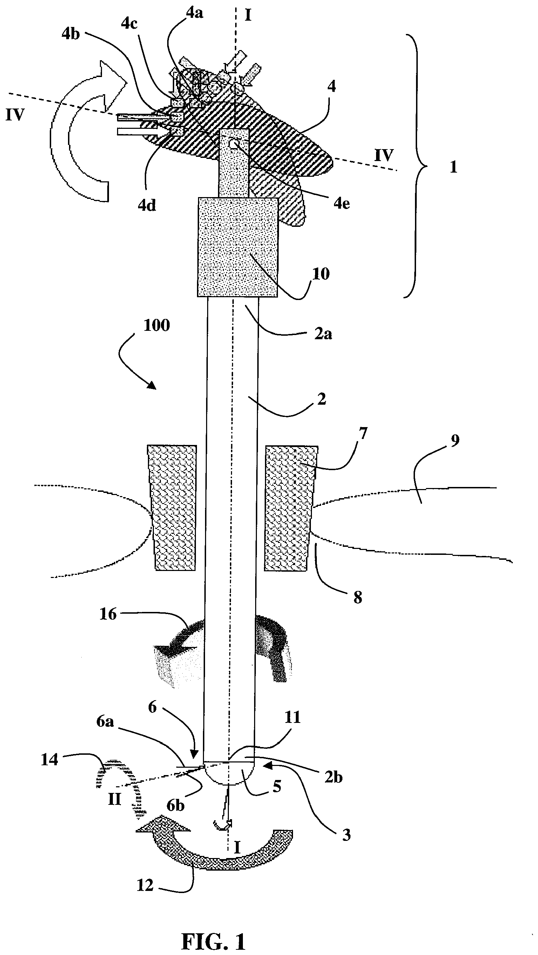

On considère tout d'abord la structure générale d'un manipulateur selon l'invention, tel qu'illustré par le mode de réalisation de la figure 1.

Dans ce mode de réalisation, le manipulateur 100 comprend de façon générale une unité de commande 1, un bras de liaison 2 qui se développe le long d'un axe longitudinal I-I de bras de liaison, et une unité de travail 3.

L'unité de commande 1 est montée sur l'extrémité proximale 2a du bras de liaison 2, tandis que l'unité de travail 3 est montée sur l'extrémité distale 2b du bras de liaison 2.

L'unité de commande 1 comprend une poignée 4 apte à être tenue par une main d'un opérateur, et un corps de commande 10 contenant divers moyens d'entraînement pour produire les mouvements appropriés dans l'unité de travail 3.

De préférence, l'unité de commande 1 est équilibrée autour de l'axe longitudinal I-I de bras de liaison, c'est-à-dire que son centre de gravité est sensiblement centré sur ledit axe longitudinal I-I de bras de liaison.

L'unité de travail 3 comporte un support d'outils 5 apte à supporter un outil 6. Sur la figure, on a illustré à titre d'exemple un outil 6 en forme de pince à deux branches 6a et 6b. D'autres formes d'outil sont naturellement possibles, par exemple un simple crochet.

La poignée 4 comporte au moins un organe de commande. Dans l'exemple décrit, la poignée 4 comporte un premier organe de commande 4a, un second organe de commande 4b, un troisième organe de commande 4c, et un organe de commande de pince 4d.

Comme dans les dispositifs connus, le bras de liaison 2 passe dans un trocart 7 assurant la liaison et le passage dans une ouverture 8 pratiquée dans la paroi corporelle 9 d'un patient. L'unité de commande 1 reste à l'extérieur du corps du patient, tandis que l'unité de travail 3 pénètre à l'intérieur du corps du patient pour atteindre la zone d'intervention où l'outil 6 doit exécuter des mouvements pilotés depuis l'unité de commande 1.

Dans le mode de réalisation illustré sur la figure 1, le support d'outils 5 et l'outil 6 peuvent être actionnés, depuis l'unité de commande 1, pour réaliser un ou plusieurs mouvements.

Par exemple, la sollicitation du premier organe de commande 4a de la poignée 4 commande un actionneur d'inclinaison logé dans le corps de commande 10 qui lui-même entraîne le support d'outils 5 en un mouvement d'inclinaison 12 autour d'un axe transversal d'inclinaison 11. Ainsi, le support d'outils 5 et l'outil 6 peuvent prendre une inclinaison dans laquelle ils sont orientés selon une direction d'inclinaison II.

Par la sollicitation du second organe de commande 4b de la poignée 4, on commande un actionneur de rotation propre logé dans le corps de commande 10, qui entraîne le support d'outils 5 en un mouvement de rotation propre 14 autour de la direction d'inclinaison II.

Par la sollicitation du troisième organe de commande 4c de la poignée 4, on commande un actionneur de rotation de bras logé dans le corps de commande 10, lequel produit un mouvement d'orientation 16 par lequel on oriente la direction d'inclinaison II autour de l'axe longitudinal I-I du bras de liaison 2.

Par l'actionnement de l'organe de commande de pince 4d de la poignée 4, on pilote un actionneur de pince pour obtenir soit l'ouverture soit la fermeture de la pince 6.

Chacun des actionneurs peut être de type moteur électrique, vérin hydraulique ou pneumatique, transmission mécanique. Les actionneurs à moteur électrique pourront être préférés, notamment pour la simplicité d'alimentation, la souplesse de fonctionnement et de commande.

La poignée 4 comporte un corps allongé le long d'un axe longitudinal de poignée IV-IV, et est articulée, dans l'unité de commande 1, autour d'un premier axe d'articulation 4e.

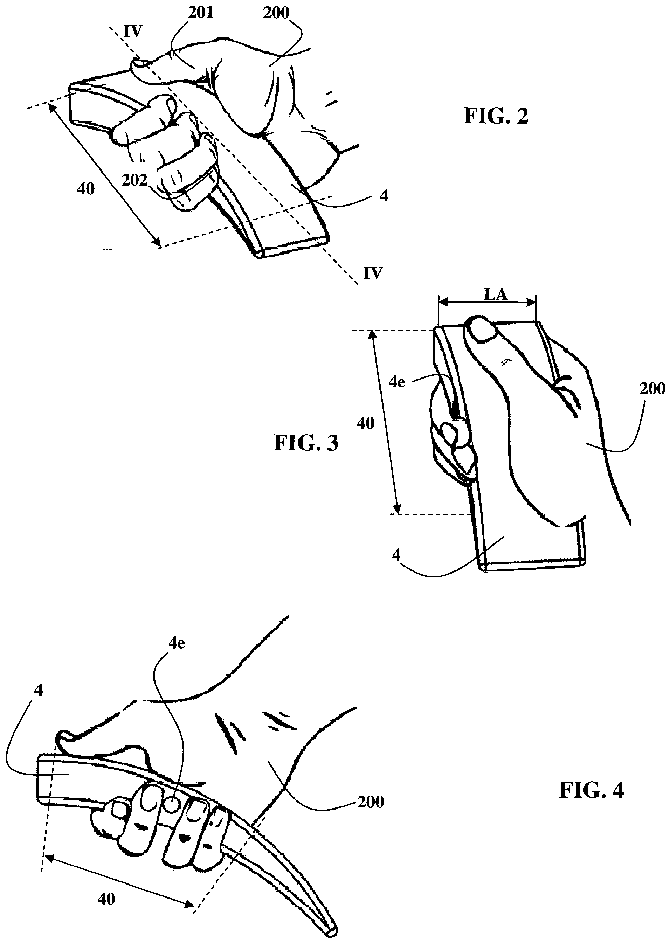

On considère maintenant les figures 2 à 4. La poignée 4 est figurée sous une forme un peu différente de celle de la figure 1, constituant encore un corps de poignée allongé le long d'un axe longitudinal de poignée IV-IV. Une main 200 se positionne normalement sur la poignée avec le pouce en opposition des autres doigts pour entourer la surface latérale de la poignée selon un tronçon de préhension 40. En pratique, la poignée 4 présente une largeur LA (figure 3) suffisamment faible pour que la poignée 4 puisse tenir dans la paume d'une main 200, avec le pouce 201 en opposition des autres doigts 202 pour entourer sa surface latérale selon le tronçon de préhension 40. La longueur du tronçon de préhension 40 est d'environ 10 cm.

La poignée 4 est articulée, dans l'unité de commande 1, autour d'un premier axe d'articulation 4e transversal par rapport à son axe longitudinal de poignée IV-IV.

On distingue, sur les figures 3 et 4, la position du premier axe d'articulation 4e transversal le long de l'axe longitudinal de poignée IV-IV.

Comme on le voit, le premier axe d'articulation 4e de la poignée 4, est en position intermédiaire le long de l'axe longitudinal de poignée IV-IV, de sorte que le tronçon de préhension 40 s'étend de part et d'autre du premier axe d'articulation 4e de la poignée 4. Dans la réalisation illustrée sur les figures 2 à 4, le premier axe d'articulation 4e est en réalité en une position intermédiaire équilibrée le long du tronçon de préhension 40 de la poignée 4, c'est-à-dire que le premier axe d'articulation 4e est sensiblement à mi-longueur du tronçon de préhension 40.

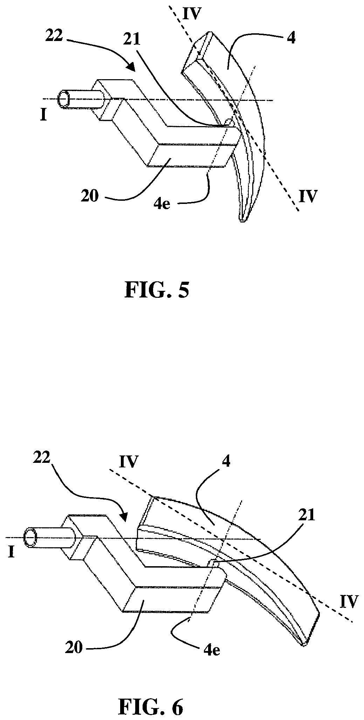

On considère maintenant un premier mode de réalisation de l'articulation de la poignée 4 dans l'unité de commande tel que représenté sur les figures 5 et 6.

On distingue, sur ces figures, l'axe longitudinal de bras I-I, l'axe longitudinal de poignée IV-IV, et le premier axe d'articulation 4e. Le premier axe d'articulation 4e est sécant avec l'axe longitudinal I-I du bras de liaison 2, et il est oblique par rapport audit axe longitudinal I-I du bras de liaison 2.

De même, l'axe longitudinal de poignée IV-IV est sécant avec l'axe longitudinal I-I du bras de liaison 2.

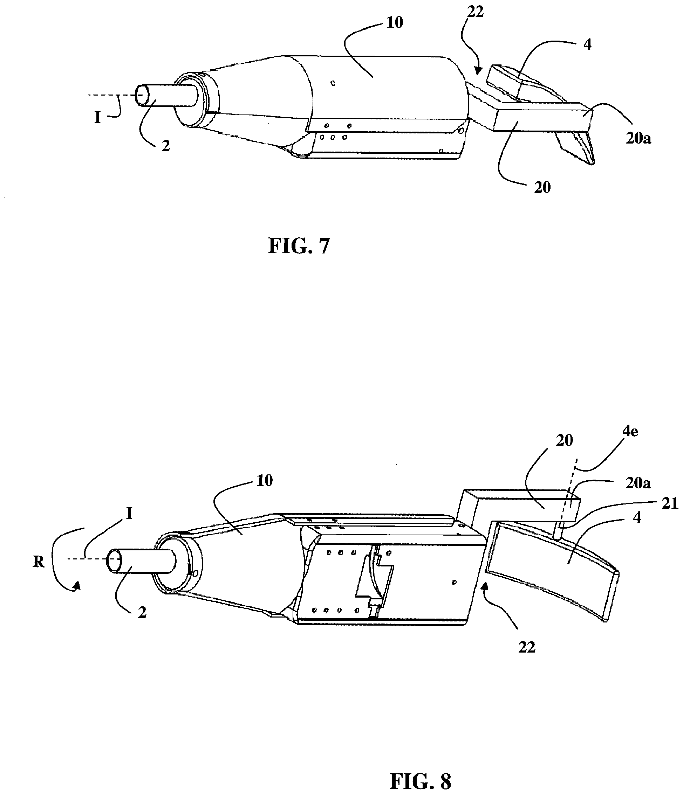

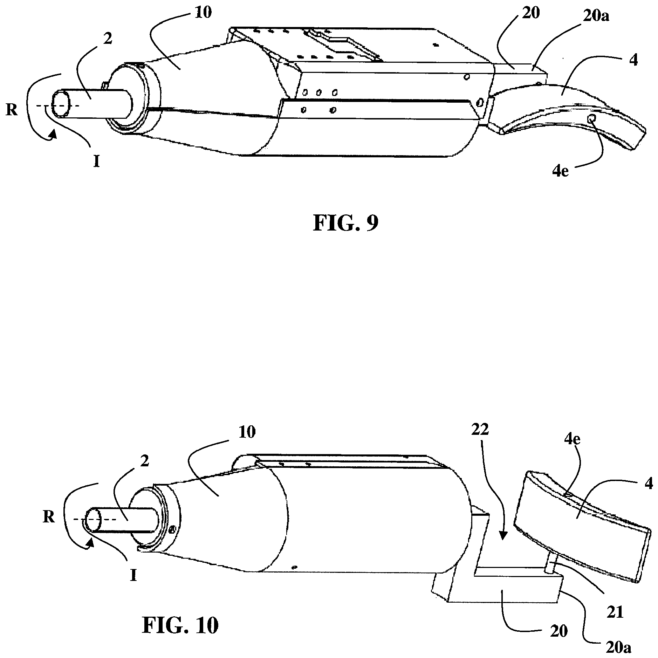

Dans la réalisation illustrée sur les figures 7 à 10, le premier axe d'articulation 4e est sensiblement perpendiculaire à l'axe longitudinal I-I du bras de liaison 2.

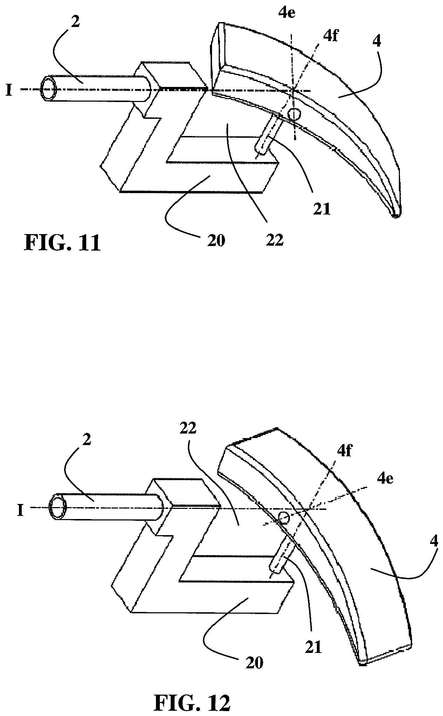

Dans la réalisation illustrée sur les figures 11 à 13, la poignée 4 est articulée selon deux axes d'articulation 4e et 4f perpendiculaires et concourants avec l'axe longitudinal I-I du bras de liaison 2.

Dans tous les modes de réalisation des figures 5 à 13, l'unité de commande 1 comprend un bras proximal longitudinal 20, déporté latéralement, à l'écart de l'axe longitudinal I-I du bras de liaison 2, et portant à son extrémité proximale 20a un bras transversal 21 sur lequel est articulée la poignée 4. Les bras 20 et 21 définissent un espace libre 22 autour de la poignée 4, pour le libre passage des doigts de la main tenant la poignée pendant les mouvements de celle-ci autour du premier axe d'articulation 4e.

On comprend que la capacité de pivotement de la poignée 4 permet de tenir la poignée dans des positions limitant l'angulation de l'articulation du poignet de la main de l'utilisateur, tout en donnant au manipulateur des orientations différentes qui correspondent à la position désirée de l'outil 6.

La manipulation des organes de commande 4a-4d permet d'actionner le support d'outils 5 et l'outil 6 sans affecter la tenue du manipulateur par la poignée 4, assurant ainsi un bon découplage entre la tenue du manipulateur et l'actionnement de l'outil 6 et du support d'outils 5.

Les figures 7 à 10 illustrent un effet intéressant de la poignée articulée et équilibrée selon l'invention, à savoir la commande d'une rotation axiale R du manipulateur par les seuls couples de rotation appliqués par la main de l'opérateur tenant la poignée 4, sans que le pouce et des doigts opposés ne quittent la poignée. Par le fait que l'unité de commande 1 est équilibrée autour de l'axe longitudinal I-I de bras, la rotation axiale R est régulière et parfaitement maîtrisée par la seule manoeuvre de la poignée 4 par laquelle la main de l'opérateur porte simultanément le manipulateur.

Les mouvements relatifs de la poignée 4 par rapport à l'unité de commande 1 pour réaliser la rotation axiale R n'affectent pas la position relative de l'outil 6 et/ou du support d'outils 5 par rapport au bras de liaison 2.

Dans tous les modes de réalisation qui ont été décrits, on peut en outre prévoir des moyens de blocage de la poignée 4 en rotation autour du ou des axes d'articulation 4e. Ce blocage peut être réalisé par tous moyens connus de l'homme du métier, par exemple par des moyens de serrage autour de l'axe d'articulation 4e.

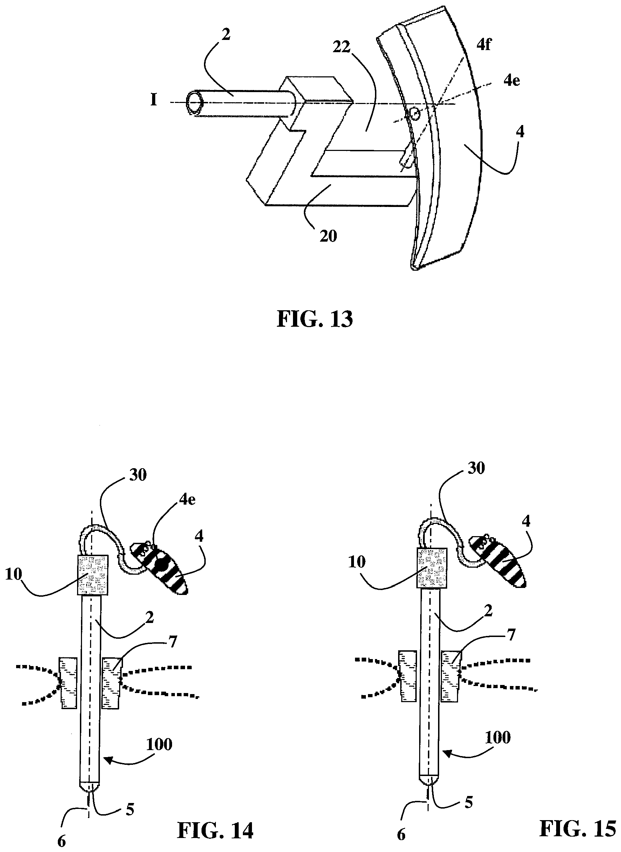

On considère maintenant la figure 14, qui illustre un manipulateur dans lequel, entre l'articulation 4e de la poignée 4 et le corps de commande 10, on prévoit un bras de positionnement 30.

Dans cette réalisation, le bras de positionnement 30 est une tige semi-rigide de 5 à 30 cm de long, déformable sous des forces de flexion ou de torsion plus importantes que les seules forces produites par le poids du manipulateur 100 et la résistance des tissus traités par le manipulateur 100.

En pratique, pour un manipulateur ayant une masse d'environ 0,5 kg, on prévoit une tige semi-rigide et qui est déformable sous l'action d'une force de 10 à 50 N, et qui conserve ensuite sa forme. De la sorte, l'utilisateur peut porter le manipulateur 100 par la poignée 4 sans déformer le bras 30.

Grâce à la capacité de déformation du bras de positionnement 30 sous l'action de forces supérieures, l'utilisateur peut conformer le bras de façon à lui donner une forme permettant la préhension de la poignée 4 en toutes positions désirées, par exemple en une position déportée. On réduit ainsi la fatigue du poignet, du coude et de l'épaule de l'opérateur, dans certaines conditions opératoires dans lesquelles le manipulateur 100 doit être déporté à l'écart du corps de l'opérateur.

La déformation et le blocage en forme du bras de positionnement 30 peuvent être réalisés en utilisant des forces hydrauliques, pneumatiques ou mécaniques, y compris les frottements. A titre d'exemple, le bras de positionnement 30 déformable peut être une tige polyarticulée, ayant une pluralité de segments articulés les uns à la suite des autres, chaque articulation étant freinée par des organes en frottement avec indexation éventuelle.

La figure 16 illustre une variante selon laquelle le bras de positionnement 30 est une tige rigide articulée sur le corps de commande 10 ou sur le bras de liaison 2 par une articulation 31 d'axe transversal munie de moyens de freinage ou d'indexage. Par exemple, l'articulation comprend au moins deux rondelles ondulées appliquées axialement l'une contre l'autre par des moyens élastiques et dont les ondulations tendent à s'engager l'une sur l'autre. La poignée 4 est articulée selon un premier axe d'articulation 4e en bout du bras de positionnement 30.

Une première invention indépendante peut consister dans l'utilisation d'un bras de positionnement de type tige semi-rigide tel que décrit ci-dessus en relation avec la figure 14, qui donne une grande capacité de modification de positionnement et d'orientation d'une poignée par rapport au reste du manipulateur. La poignée 4 est articulée en bout du bras de positionnement 30.

La figure 15 illustre une autre possibilité, dans laquelle la poignée 4 est fixe en bout d'un bras de positionnement 30 de type tige semi-rigide qui la relie au corps de commande 10 et au bras de liaison 2 dont l'extrémité distale porte le support d'outils 5 et l'outil 6.

Une seconde invention indépendante peut consister dans l'utilisation d'un bras de positionnement de type tige rigide articulée à l'extrémité du bras de liaison, comme décrit ci-dessus en relation avec la figure 16. Dans ce cas, l'ergonomie nécessite de prévoir au moins un organe de commande, monté sur la poignée, et commandant un actionneur qui entraîne le support d'outils 5 en un mouvement d'inclinaison par rapport à l'axe longitudinal du bras de liaison 2.

La figure 17 illustre une autre possibilité, dans laquelle la poignée 4 est fixe en bout du bras de positionnement 30 de type tige rigide articulée, et le support d'outils 5 est commandé en orientation 12 par un des organes de commande 4a de la poignée 4.

Pour le reste, ces dispositifs des figures 14 à 17 peuvent reprendre les moyens précédemment illustrés en relation avec la figure 1.

Les figures 18 et 19 illustrent l'effet ergonomique de la présence d'un bras de positionnement 30 dans le cas d'une manipulation du manipulateur en position excentrée : voir sur la figure 18 la position écartée et fatigante du bras droit 301 de l'opérateur 300 qui porte un manipulateur 100a de l'art antérieur ; et voir sur la figure 19 la position naturelle rapprochée et confortable du bras droit 301 de l'opérateur 300 qui porte un manipulateur 100 à bras de positionnement 30 selon l'invention.

La présente invention n'est pas limitée aux modes de réalisation qui ont été explicitement décrits, mais elle en inclut les diverses variantes et généralisations contenues dans le domaine des revendications ci-après.

## Revendications

1. Manipulateur comprenant :
- une unité de commande (1), ayant une poignée (4) apte à être tenue par une main,
- au moins un organe de commande (4a-4d), monté sur la poignée (4), et apte à être sollicité par au moins un doigt de la main tenant la poignée (4),
- un bras de liaison (2), se développant le long d'un axe longitudinal (I-I) de bras, ayant une extrémité proximale (2a) dans laquelle est montée l'unité de commande (1), et ayant une extrémité distale (2b),
- une unité de travail (3), montée sur l'extrémité distale (2b) du bras de liaison (2), comportant un support d'outils (5) apte à supporter un outil (6), et actionnée par ledit au moins un organe de commande (4a-4d),
- la poignée (4) étant allongée le long d'un axe longitudinal de poignée (IV-IV) et étant conformée pour être tenue dans la paume de la main (200) avec le pouce (201) en opposition des autres doigts (202) pour entourer sa surface latérale selon un tronçon de préhension (40),
- la poignée (4) étant articulée, dans l'unité de commande (1), selon au moins un degré de liberté de rotation autour d'un premier axe d'articulation (4e) transversal par rapport à son axe longitudinal de poignée (IV-IV),
- le premier axe d'articulation (4e) de la poignée (4) étant en position intermédiaire le long de l'axe longitudinal de poignée (IV-IV), de sorte que le tronçon de préhension (40) s'étend de part et d'autre du premier axe d'articulation (4e) de la poignée (4),
- le premier axe d'articulation (4e) étant sécant avec l'axe longitudinal (I-I) du bras de liaison (2),
- l'axe longitudinal de poignée (IV-IV) est sécant avec l'axe longitudinal (I-I) du bras de liaison (2),
- la poignée (4) est fixe en rotation axiale autour de son axe longitudinal (IV-IV) par rapport à l'unité de commande (1),
- le manipulateur est dépourvu de tout moyen de couplage direct ou indirect entre les mouvements relatifs de la poignée (4) par rapport à l'unité de commande (1) et les mouvements relatifs du support d'outils (5) et de l'outil (6) par rapport au bras de liaison (2),
- les mouvements relatifs du support d'outils (5) et de l'outil (6) par rapport au bras de liaison (2) sont produits par des actionneurs logés dans un corps de commande (10) de l'unité de commande (1) et commandés par ledit au moins un organe de commande (4a-4d).

2. Manipulateur selon la revendication 1, **caractérisé en ce que** le premier axe d'articulation (4e) est en position intermédiaire équilibrée le long du tronçon de préhension (40) de la poignée (4).

3. Manipulateur selon l'une des revendications 1 ou 2, **caractérisé en ce que** la poignée (4) est en outre articulée dans l'unité de commande (1) selon un second degré de liberté de rotation autour d'un second axe d'articulation (4f).

4. Manipulateur selon la revendication 3, **caractérisé en ce que** les axes d'articulation (4e, 4f) sont concourants avec l'axe longitudinal (I-I) du bras (2).

5. Manipulateur selon l'une quelconque des revendications 1 à 4, **caractérisé en ce qu'**il comprend en outre des moyens de blocage de la poignée (4) en rotation autour du ou des axes d'articulation (4e, 4f).

6. Manipulateur selon l'une quelconque des revendications 1 à 5, **caractérisé en ce que** l'actionnement d'un premier organe de commande (4a) commande un actionneur d'inclinaison logé dans un corps de commande (10), qui entraîne le support d'outils (5) en un mouvement d'inclinaison (12).

7. Manipulateur selon l'une quelconque des revendications 1 à 6, **caractérisé en ce que** la poignée (4) est articulée selon un seul degré de liberté de rotation, autour du premier axe d'articulation (4e).

8. Manipulateur selon la revendication 7, **caractérisé en ce que** le premier axe d'articulation (4e) est oblique par rapport à l'axe longitudinal (I-I) du bras de liaison (2).

9. Manipulateur selon l'une quelconque des revendications 1 à 8, **caractérisé en ce que** le centre de gravité de l'unité de commande (1) est sensiblement centré sur l'axe longitudinal de bras (I-I).

## Patentansprüche

1. Manipulator umfassend:
- eine Steuereinheit (1) aufweisend einen Griff (4), der eingerichtet ist, in einer Hand gehalten zu werden,
- wenigstens ein Steuerglied (4a-4d), das auf dem Griff (4) befestigt und eingerichtet ist, durch wenigstens einen Finger der den Griff (4) haltenden Hand belastet zu werden,
- einen Verbindungsarm (2), der sich entlang eines Armes entlang einer Längsachse (I-I) erstreckt, der ein nahes Ende (2a) aufweist, in dem die Steuereinheit (1) befestigt ist, und der ein fernes Ende (2b) aufweist,
- eine Betriebseinheit (3), die am entfernten Ende (2b) des Verbindungsarmes (2) befestigt ist, eine Werkzeugaufnahme (5) enthält, die eingerichtet ist, ein Werkzeug (6) zu tragen, und die durch das genannte wenigstens eine Steuerglied (4a-4d) bewegt wird,
- der Griff (4) entlang einer Grifflängsachse (IV-IV) gestreckt ist und ausgebildet ist, in der Handfläche (200) mit dem Daumen (201) gegenüberliegend den anderen Fingern (202) gehalten zu werden, um seine äußere Oberfläche in einem Halteabschnitt (40) zu umschließen,
- der Griff (4) in der Steuereinheit (1) mit wenigstens einem Drehfreiheitsgrad um eine erste Gelenkachse (4e) quer zu seiner Grifflängsachse (IV-IV) ausgestattet ist,
- die erste Gelenkachse (4e) des Griffs (4) sich in einer Zwischenposition entlang der Grifflängsachse (IV-IV) befindet mit dem Ergebnis, dass sich der Halteabschnitt (40) von beiden Seiten der ersten Gelenkachse (4e) des Griffs (4) erstreckt,
- die erste Gelenkachse (4e) die Längsachse (I-I) des Verbindungsarms (2) schneidet,
- die Grifflängsachse (IV-IV) die Längsachse (I-I) des Verbindungsarms (2) schneidet,
- der Griff (4) gegenüber axialer Drehung um seine Längsachse (IV-IV) relativ zur Steuereinheit (1) fixiert ist,
- der Manipulator frei von direkten oder indirekten Kopplungsmitteln zwischen der Bewegung des Griffs (4) relativ zur Steuereinheit (1) und der Bewegung der Werkzeugaufnahme (5) und dem Werkzeug (6) relativ zum Verbindungsarm (2) ist,
- die Bewegung der Werkzeugaufnahme (5) und dem Werkzeug (6) relativ zum Verbindungsarm (2) mittels eines Stellgliedes erreicht wird, das in einem Steuerkörper (10) der Steuereinheit (1) aufgenommen ist und über das genannte wenigstens eine Steuerglied (4a-4d) gesteuert wird.

2. Manipulator nach Anspruch 1, **dadurch gekennzeichnet, dass** sich die erste Gelenkachse (4e) in einer ausgeglichenen Zwischenposition entlang des Halteabschnittes (40) des Griffs (4) befindet.

3. Manipulator nach einem der Ansprüche 1 oder 2, **dadurch gekennzeichnet, dass** der Griff (4) ferner in der Steuereinheit (1) mit einem zweiten Drehfreiheitsgrad um eine zweite Gelenkachse (4f) ausgestattet ist.

4. Manipulator nach Anspruch 3, **dadurch gekennzeichnet, dass** die Gelenkachsen (4e, 4f) die Längsachse (I-I) des Arms (2) schneiden.

5. Manipulator nach einem der Ansprüche 1 bis 4, **dadurch gekennzeichnet, dass** er ferner Mittel zum Blockieren des Griffs (4) gegen eine Drehung um die Gelenkachse oder -achsen (4e, 4f) umfasst.

6. Manipulator nach einem der Ansprüche 1 bis 5, **dadurch gekennzeichnet, dass** die Bewegung eines ersten Steuergliedes (4a) ein Neigestellglied steuert, das in einem Steuerkörper (10) aufgenommen ist, welcher die Werkzeugaufnahme (5) in einer Neigebewegung (12) steuert.

7. Manipulator nach einem der Ansprüche 1 bis 6, **dadurch gekennzeichnet, dass** der Griff (4) mit nur einem Drehfreiheitsgrad um die erste Gelenkachse (4e) ausgestattet ist.

8. Manipulator nach Anspruch 7, **dadurch gekennzeichnet, dass** die erste Gelenkachse (4e) winklig zur Längsachse (I-I) des Verbindungsarms (2) steht.

9. Manipulator nach einem der Ansprüche 1 bis 8, **dadurch gekennzeichnet, dass** der Schwerpunkt der Steuereinheit (1) im Wesentlichen mittig auf der Armlängsachse (I-I) liegt.

## Claims

1. Manipulator comprising:
- a control unit (1), having a handle (4) adapted to be held in one hand,
- at least one control member (4a-4d), mounted on the handle (4), and adapted to be loaded by at least one finger of the hand holding the handle (4),
- a connecting arm (2), extending along an arm longitudinal axis (I-I), having a proximal end (2a) in which the control unit (1) is mounted, and having a distal end (2b),
- a working unit (3), mounted on the distal end (2b) of the connecting arm (2), including a tool support (5) adapted to support a tool (6), and actuated by said at least one control member (4a-4d),
- the handle (4) being elongate along a longitudinal axis of the handle (IV-IV) and being configured to be held in the palm of the hand (200) with the thumb (201) opposed to the other fingers (202) to surround its lateral surface in a holding section (40),
- the handle (4) being articulated, in the control unit (1), with at least one degree of freedom in rotation about a first articulation axis (4e) transverse with respect to its longitudinal axis of the handle (IV-IV),
- the first articulation axis (4e) of the handle (4) being in an intermediate position along the longitudinal axis of the handle (IV-IV), with the result that the holding section (40) extends either side of the first articulation axis (4e) of the handle (4),
- the first articulation axis (4e) intersecting the longitudinal axis (I-I) of the connecting arm (2),
- the longitudinal axis of the handle (IV-IV) intersects the longitudinal axis (I-I) of the connecting arm (2),
- the handle (4) is fixed against axial rotation about its longitudinal axis (IV-IV) with respect to the control unit (1),
- the manipulator is devoid of any direct or indirect coupling means between the relative movements of the handle (4) with respect to the control unit (1) and the relative movements of the tool support (5) and of the tool (6) with respect to the connecting arm (2),
- the relative movements of the tool support (5) and of the tool (6) with respect to the connecting arm (2) are produced by actuators accommodated in a control body (10) of the control unit (1) and controlled by said at least one control member (4a-4d).

2. Manipulator according to claim 1, **characterized in that** the first articulation axis (4e) is in a balanced intermediate position along the holding section (40) of the handle (4).

3. Manipulator according to either of claims 1 or 2, **characterized in that** the handle (4) is additionally articulated in the control unit (1) with a second degree of freedom in rotation about a second articulation axis (4f) .

4. Manipulator according to claim 3, **characterized in that** the articulation axes (4e, 4f) intersect the longitudinal axis (I-I) of the arm (2).

5. Manipulator according to any of claims 1 to 4, **characterized in that** it additionally comprises means for locking in the handle (4) against rotation about the articulation axis or axes (4e, 4f).

6. Manipulator according to any of claims 1 to 5, **characterized in that** the actuation of a first control member (4a) controls an inclination actuator accommodated in a control body (10) that drives the tool support (5) in an inclination movement (12).

7. Manipulator according to any of claims 1 to 6, **characterized in that** the handle (4) is articulated with only one degree of freedom in rotation about the first articulation axis (4e).

8. Manipulator according to claim 7, **characterized in that** the first articulation axis (4e) is oblique with respect to the longitudinal axis (I-I) of the connecting arm (2).

9. Manipulator according to any of claims 1 to 8, **characterized in that** the centre of gravity of the control unit (1) is substantially centred on the longitudinal axis of the arm (I-I).
